# FASCICULE DE BREVET EUROPEEN

(11) **EP 4 149 426 B1**
(45) Date de publication et mention de la délivrance du brevet: **28.05.2025**
(21) Numéro de dépôt: 21731241.2
(22) Date de dépôt: 11.05.2021
(51) Int. Cl.: A61K 9/00, A61K 9/20

(54) **COMPRIME MUCOADHESIF POUR LE TRAITEMENT DES INFECTIONS FONGIQUES OROPHARYNGEES**
MUKOADHÄSIVE TABLETTE ZUR BEHANDLUNG VON OROPHARYNGEALEN PILZINFEKTIONEN
MUCOADHESIVE TABLET FOR THE TREATMENT OF OROPHARYNGEAL FUNGAL INFECTIONS

(30) Priorité: 12.05.2020 FR 2004657
(43) Date de publication de la demande: 22.03.2023
(73) Titulaire: Vectans Pharma, 92213 Saint-Cloud Cedex (FR)
(72) Inventeur: COSTANTINI, Dominique, 75014 PARIS (FR); ATTALI, Pierre, 94300 VINCENNES (FR); THERON, Jean, 78000 VERSAILLES (FR)
(74) Mandataire: Cabinet Becker et Associés
(86) Numéro de dépôt international: PCT/FR2021/050805
(87) Numéro de publication internationale: WO 2021/229170

(56) Documents cités:
- FR-A1- 2 827 517
- US-A1- 2009 137 477
- SUDHAKAR Y ET AL: "Buccal bioadhesive drug delivery - A promising option for orally less efficient drugs", JOURNAL OF CONTROLLED RELEASE, ELSEVIER, AMSTERDAM, NL, vol. 114, no. 1, 10 August 2006 (2006-08-10), pages 15 - 40, XP024957569, ISSN: 0168-3659, [retrieved on 20060810], DOI: 10.1016/J.JCONREL.2006.04.012

## Description

### DOMAINE TECHNIQUE

La présente invention se rapporte au domaine thérapeutique, plus précisément aux compositions mucoadhésives destinées au traitement des infections oropharyngées telles que les candidoses oropharyngées.

### ARRIERE PLAN TECHNOLOGIQUE

La candidose est une maladie fongique (aussi appelée mycose) causée par les levures de genre *Candida.* Les levures du genre *Candida* sont saprophytes, c'est-à-dire qu'elles font partie de la flore commensale. Cependant, sous certaines conditions, elles se transforment en une forme filamenteuse pathogène capable d'adhérer et de coloniser les muqueuses. On compte environ 23 espèces de Candida susceptibles d'être pathogènes pour l'homme, notamment *Candida albicans* qui est l'agent pathogène principal responsable des mycoses oropharyngées. D'autres espèces de Candida peuvent être impliquées telles *C*. *tropicalis, C. glabrata, C. kefyr, C. guilliermondii, C. krusei, C. lusitaniae et C. parapsilosis.*

A l'heure actuelle, il existe plusieurs médicaments locaux pour le traitement de candidoses oropharyngées. Ces médicaments se présentent sous la forme de gel tel que le Daktarin^{®} (miconazole), de suspension buvable ou bain de bouche comme le Fungizone^{®} (amphotéricine B) ou la Mycostatine^{®} (nystatine), de comprimés pour l'administration gingivale tel que le Loramyc^{®} (miconazole).

Cependant, les traitements médicaux antifongiques sont parfois insuffisamment efficaces pour guérir la candidose oropharyngée et les récidives imposent de retraiter les patients avec d'autres classes de médicaments pour prévenir des infections systémiques. Le contenu élevé en sucres des formulations buvables ou des gels associé à des concentrations locales, au site de l'infection, trop fluctuantes ou trop transitoires entraîne l'émergence de résistance et favorise les rechutes des candidoses oropharyngées.

Certaines candidoses oropharyngées ont une résistance primaire ou secondaire aux médicaments antifongiques. La résistance peut être liée à de multiples mécanismes, principalement des mutations ou une surexpression de l'enzyme cible et de certaines protéines des pompes impliquées dans l'efflux des antifongiques. De multiples mécanismes coexistent fréquemment et des phénomènes de résistance croisée entre différents antifongiques ne sont pas rares. A côté de ces mécanismes de résistance, il est signalé depuis quelques années la capacité qu'ont les levures à former de biofilms qui les protègent des facteurs externes tels que le système immunitaire de l'hôte ou des médicaments antifongiques.

Les biofilms sont des structures complexes tridimensionnelles composées d'un réseau dense de levures, d'hyphes et de pseudo-hyphes entouré d'une matrice extracellulaire de polysaccharides.

Les candidoses oropharyngées à biofilm, notamment buccales, ont une étiologie multifactorielle. Il existe plusieurs facteurs favorisant le développement des candidoses oropharyngées avec biofilm tels que le port d'un dispositif médical, l'âge, l'hyposialie physiologique ou iatrogène, les traumatismes buccaux, la prise massive d'antibiotique ou de corticostéroïdes, certaines maladies systémiques comme le diabète et le cancer, les sujets immunodéprimés, par exemple souffrant du VIH ou ayant subi une radiothérapie ou une chimiothérapie.

Concernant les dispositifs médicaux, plusieurs études ont montré qu'ils constituent des surfaces propices au développement des biofilms *à Candida.* Ainsi, les prothèses dentaires, dentiers, systèmes d'orthodonties et autres dispositifs médicaux dentaires peuvent être à l'origine de candidoses buccales. En effet, les espèces de *Candida* adhèrent très facilement à la surface de ces dispositifs médicaux, ce qui facilite la formation d'un biofilm capable de coloniser la muqueuse adjacente, puis l'ensemble de la bouche et la langue. Cette infection peut conduire à une stomatite candidosique aïgue (par exemple comme dans le cas des stomatites *à Candida* associées au port d'un dentier - Candida-associated denture stomatitis (CaDs)) caractérisée par une inflammation, un érythème chronique et/ou un œdème de la muqueuse buccale rendant la prise alimentaire particulièrement douloureuse et pouvant entrainer des complications systémiques graves chez les personnes fragiles.

Plusieurs études *in vitro* ont montré que les biofilms à *Candida* présentent une résistance accrue aux antifongiques par rapport aux formes planctoniques. Cette résistance a été observée vis-à-vis de différentes classes d'antifongiques, par exemple avec les polyènes comme l'amphotéricine B ou encore les antifongiques azolés tels que le fluconazole, l'itraconazole, le miconazole et le voriconazole. Ces études sont concordantes avec l'expérience clinique qui montre que les candidoses oropharyngées à biofilm répondent mal aux traitements antifongiques et que les rechutes sont fréquentes avec un risque élevé de dissémination systémique (Gebremedhin et al., Journal of Physiology and Pharmacology, 2014, 65,4,593-600).

Le document US 2009/137477 A1 divulgue un comprimé mucoadhésif buccal a libération prolongée comprenant 30% à 50% en poids de miconazole, 18 à 28% de protéines de lait, un surfactant, et de l'HPMC. Le document, dans le tableau 1, divulgue une composition comprenant 43% de miconazole, 18% de HPMC, 24% de protéine de lait, 8% d'amidon de maïs, 0,34% de lactose, 2% de stéarate de magnésium et de talc, 4,5% de surfactant.

Le document FR 2 827 517 A1 divulgue aussi un comprimé mucoadhésif pour libération prolongée de miconazole.

Il existe donc à l'heure actuelle un besoin de nouvelles stratégies de prise en charge des infections oropharyngées à biofilm.

### RESUME DE L'INVENTION

Selon un premier aspect, l'invention a pour objet un comprimé mucoadhésif buccal à libération prolongée comprenant de 25 à 150 mg, de préférence de 35 à 135 mg, de miconazole en tant que principe actif en association avec un concentré de protéines de lait et de l'hydroxypropyl méthylcellulose (HPMC) en tant qu'excipients caractérisé en ce que :
- le comprimé présente une dureté supérieure à 20 N et une adhésivité supérieure à 100 g,
- le comprimé est obtenu par un procédé de granulation, de préférence par voie humide,
- le comprimé comprend des grains primaires comprenant le miconazole, le concentré de protéines de lait et une partie de l'HPMC, la partie restante de l'HPMC étant en phase externe des grains primaires et représentant de 45% à 55% en poids du poids total de l'HPMC présent dans le comprimé.

Dans certains modes de réalisation, le comprimé selon l'invention présente un « rapport dureté/adhésivité » inférieur à 0.4, de préférence allant de 0,04 à 0,4. L'adhésivité, c'est-à-dire la force de bioadhésion du comprimé à la muqueuse gingivale, et la dureté sont déterminés par des méthodes classiques, telles qu'illustrées ci-après.

Ledit comprimé peut comprendre en outre un surfactant, de préférence un sel de dodécyl sulfate, en phase externe.

Le comprimé selon l'invention peut également comprendre un désintégrant tel que l'amidon de maïs présent dans les grains primaires, et un ou plusieurs lubrifiants tels que le talc et le stéarate de magnésium présents en phase externe.

Le comprimé mucoadhésif présente, de préférence, un profil de libération *in vitro* suivant:
- De 20% à 45% du miconazole présent dans le comprimé est libéré en 4 heures,
- De 60 % à 85% du miconazole présent dans le comprimé est libéré en 8 heures, et
- Au moins 85 % du miconazole présent dans le comprimé est libéré en 24h,
le profil de dissolution *in vitro* étant déterminé par un test de dissolution de préférence tel que défini dans la pharmacopée US (USP), chapitre 711 dans les conditions suivantes:
- dispositif de dissolution à paniers : USP Dissolution Apparatus 1
- milieu de dissolution : SDS à 0,5% dans de l'eau purifiée à pH ajusté à 6.5±0,5
- Volume de dissolution: 1000 mL,
- température : 37±0,5°C et
- vitesse de rotation : 60 rpm.

Dans certains modes de réalisation, le comprimé mucoadhésif selon l'invention est caractérisé en ce qu'il instaure une concentration salivaire en miconazole 24h après son application sur la gencive du sujet (C₂₄ₕ) d'au moins 100 µg/mL, de préférence d'au moins 140 µg/mL. Selon certains modes de réalisation, le comprimé selon l'invention comprend 50 mg de miconazole et présente les propriétés pharmacocinétiques suivantes : une Cmax salivaire moyenne de 300 à 500 µg/, une Tmax salivaire moyenne d'environ 13 à 15h et une AUCinf salivaire moyenne de 2000 à 3000 µg.h/mL.

Dans d'autres modes de réalisation, le comprimé selon l'invention est caractérisé en ce que le concentré de protéines de lait se présente sous la forme d'une poudre atomisée et a une teneur en protéine d'au moins 85%, de préférence au moins 87% en poids et une teneur en calcium d'au moins 1% en poids, de préférence d'environ 2,2% en poids et/ou l'HPMC a une viscosité apparente dans l'eau à 20°C de 11 000 à 22 000 cPs. De plus, le comprimé mucoadhésif selon l'invention peut comprendre :
- de 35% à 50% de miconazole,
- de 10% à 25 %, de préférence de 15% à 20% d'HPMC
- de 15% à 30%, de préférence de 20% à 26% d'un concentré de protéines de lait,
- de 5% à 15%, de préférence de 6% à 10 % d'un désintégrant, de préférence un amidon de maïs,
- de 0% à 3% de lactose, de préférence de 0,1% à 0,8% de lactose,
- de 2% à 8% de surfactant, de préférence de 3% à 6% de dodécylsulfate de sodium, et
- de 1% à 3% de lubrifiant, de préférence de 1,5% à 2,5% de magnésium stéarate et de talc, les pourcentages étant exprimés en poids par rapport au poids total du comprimé.

Typiquement, la quantité de miconazole est de 50 mg ou 100 mg dans le comprimé.

Le comprimé selon l'invention peut être obtenu par un procédé de granulation humide comprenant les étapes suivantes :
a) le mélange du miconazole avec le concentré de protéines de lait, une partie de l'HPMC et le désintégrant
b) la granulation humide du mélange de l'étape a) par ajout d'eau comprenant le lactose,
c) le séchage et le calibrage des grains obtenus à l'étape b),
d) l'ajout du reste d'HPMC, du surfactant et des lubrifiants aux grains obtenus à l'étape c), et
e) la compression et la mise en forme du mélange de l'étape d) de manière à obtenir un comprimé.

Typiquement, 45% à 55%, de préférence environ 50% en poids du poids total de l'HPMC présent dans le comprimé final sont ajoutés à l'étape a). L'étape e) est réalisée de manière à ce que la dureté du comprimé soit d'au moins 20N, de préférence de 20N à 60 N, par exemple de 25N à 50 N. Dans un mode de réalisation particulier, le comprimé selon l'invention est destiné aux sujets souffrant ou prédisposés aux infections oropharyngées à biofilm.

La présente description divulgue aussi l'utilisation d'un comprimé tel que défini ci-avant dans le traitement et la prévention d'une infection oropharyngée à biofilm chez un sujet. Ladite infection peut être une candidose buccale associée, de préférence à une ou plusieurs espèces de Candida choisies parmi *C*. *albicans, C. tropicalis, C. glabrata, C. kefyr, C. guilliermondii, C. krusei, C. lusitaniae et C. parapsilosis.* Dans certains modes de réalisation, le sujet traité présente un ou plusieurs facteurs de risque choisis parmi un âge élevé, de préférence supérieur à 75 ans, le diabète, un cancer, hyposialie physiologique ou iatrogène, une immunodépression, une malnutrition, la consommation de cigarettes, une surconsommation d'antiseptiques buccaux, le port d'un dispositif médical oral, et des traitements médicaux tels qu'une corticothérapie, notamment par inhalation, des antibiotiques, des immunosuppresseurs, une radiothérapie notamment du cou et de la tête et une chimiothérapie.

Dans un mode de réalisation particulier, le comprimé est utilisé dans le traitement et la prévention d'une infection oropharyngée à biofilm, le sujet portant un dispositif médical oral de préférence choisi parmi un dentier, une prothèse dentaire fixe ou amovible et un appareil d'orthodontie. L'infection oropharyngée à biofilm peut être, notamment, une stomatite candidosique associée au port d'un dispositif médical dentaire.

Une méthode de traitement d'une infection oropharyngée à biofilm chez un sujet, comprenant l'administration par voie buccale d'un comprimé mucoadhésif tel que défini ci-avant est aussi décrite mais ne fait pas partie de l'invention. La présente description divulgue également l'utilisation du miconazole pour la préparation d'un médicament pour le traitement d'une infection oropharyngée à biofilm, ledit médicament se présentant sous la forme d'un comprimé mucoadhésif tel que défini ci-avant.

Enfin, l'invention a pour objet un procédé de fabrication d'un comprimé mucoadhésif de miconazole selon l'invention par granulation humide comprenant les étapes suivantes :
a) le mélange du miconazole avec une partie de l'HPMC, le concentré de protéines de lait et le désintégrant
b) la granulation humide du mélange de l'étape a) par ajout d'eau comprenant le lactose,
c) le séchage et le calibrage des grains obtenus à l'étape b),
d) l'ajout du reste d'HPMC, du surfactant et des lubrifiants aux grains obtenus à l'étape c), et
e) la compression et la mise en forme du mélange de l'étape d) de manière à obtenir un comprimé,

dans lequel de 45% à 55%, de préférence environ 50% en poids du poids total de l'HPMC présent dans le comprimé final sont ajoutés à l'étape a) et
dans lequel l'étape e) est réalisée de manière à ce que la dureté du comprimé soit d'au moins 20N, de préférence de 20N à 60 N, par exemple de 25N à 50 N.

### DESCRIPTION DES FIGURES

La Figure 1 montre les profils de dissolutions in vitro du comprimé selon l'invention (forme B) et du comprimé de référence (forme A).
La Figure 2 montre l'évolution de la concentration salivaire moyenne au cours du temps pour les comprimés selon l'invention (forme B) *versus* celle obtenue le comprimé de référence (forme A).
La Figure 3 montre l'effet de la répartition de l'HPMC entre phase externe et phase interne sur la quantité de miconazole libérée in vitro à T 24h pour des comprimés obtenus avec une force de compression élevée ou une force de compression plus faible.

### DESCRIPTION DETAILLEE DE L'INVENTION

Gebremedhin *et al.* (supra) ont testé l'activité antifongique du miconazole sur un modèle de biofilm de différentes espèces de *Candida in vitro.* Gebremedhib *et al.* ont observé une activité antifongique du miconazole vis-à-vis de ces biofilms. Néanmoins, les auteurs ont montré qu'une concentration en miconazole d'environ 100 µg/ml est nécessaire pour diminuer d'au moins 50% l'activité métabolique des biofilms chez toutes les espèces testées, à savoir *C*. *albicans, C. glabrata, C. tropicalis, et C. parapsilosis.*

Sur la base de cette constatation, la Demanderesse a analysé qu'il est nécessaire d'atteindre une concentration salivaire supérieure à 100 µg/ml, de manière prolongée, pour espérer traiter les candidoses buccales à biofilm.

La demande internationale WO03/009800 décrit un comprimé mucoadhésif de miconazole comprenant notamment en tant qu'excipients des protéines de lait, de l'hydroxy propyl methyl cellulose (HPMC) et du dodecylsulfate de sodium (SDS). Ce comprimé est obtenu par un procédé de granulation humide dans lequel le miconazole est mélangé avec l'HPMC et les protéines de lait. Le mélange ainsi obtenu est granulé par une solution aqueuse contenant du lactose de manière à obtenir un grain primaire auquel sont rajoutés le SDS et d'autres excipients (lubrifiants). Après mélange, le comprimé est obtenu par compression. L'HPMC est essentiellement présent en phase interne (c'est-à-dire dans les grains primaires). La demande internationale WO03/009800 ne fournit aucune indication quant à la dureté et la bioadhésivité des comprimés obtenus mais décrit que ces comprimés présentent une Cmax salivaire de 15,07 µg/ml, une tmax salivaire de 7h et une AUC salivaire 0-12h d'environ 43 µgl.h/ml. A l'évidence, le comprimé décrit dans la demande WO03/009800 ne permet pas d'instaurer une concentration salivaire prolongée de 100 µg/ml nécessaire pour traiter efficacement les candidoses buccales à biofilm.

La Demanderesse a donc recherché à modifier la forme pharmaceutique décrite dans la demande internationale WO03/009800 de manière à obtenir, de manière stable et rapide, une concentration salivaire d'au moins 100 µg de miconazole par ml de salive chez les patients tout en conciliant un schéma posologique simple, à savoir l'administration de cette forme pharmaceutique une fois par jour.

De manière surprenante, la Demanderesse a montré qu'il était possible d'améliorer le profil de libération du miconazole en augmentant la dureté de ce comprimé.

Un tel résultat est tout à fait surprenant dans la mesure où l'homme du métier aurait attendu qu'une augmentation de la dureté altère l'intégrité du comprimé, empêche ou retarde la pénétration salivaire et donc la dissolution du principe actif, gêne également son adhésion à la muqueuse du patient. L'homme du métier aurait pu également s'attendre à ce qu'une dureté accrue du comprimé empêche la libération rapide du principe actif localement et/ou favorise une libération systémique importante du principe actif ce qui est non souhaité notamment en raison des effets secondaires associés. En d'autres termes, l'homme du métier aurait également attendu que l'augmentation de la dureté altère de manière significative le profil de libération du principe actif en diminuant sa vitesse de libération initiale et en empêchant sa libération prolongée dans le temps à cause d'un délitement incomplet du comprimé dans la cavité orale. Or, de manière surprenante, la Demanderesse a montré qu'un comprimé avec une dureté supérieure à 20 N présentait un profil de dissolution *in vitro* amélioré par rapport à un comprimé témoin, de composition similaire mais présentant une dureté d'environ 17N.

De manière surprenante, la Demanderesse a également montré que cette différence *in vitro* se traduisait *in vivo* chez l'homme par une durée d'adhésion du comprimé plus élevée (24 h versus 15h pour le comprimé de référence) et une modification nette du profil pharmacocinétique : Le comprimé mucoadhésif selon l'invention comprenant une dose de 50 mg de miconazole permet une libération prolongée du miconazole caractérisée par une Cmax salivaire de 346 µg /ml, un Tmax de 14 h, et une AUC de 2630 µg.h/mL. De manière notable, la concentration salivaire en miconazole à 24h est d'environ 150 µg/ml, ce qui est bien supérieure à la concentration déterminée par Gebremedhin et al. pour provoquer une inhibition d'au moins 50% de l'activité métabolique chez toutes les souches de Candida testées. Ces caractéristiques pharmacocinétiques sont nettement meilleures que celles observées pour le comprimé témoin et celles du comprimé décrit dans la demande WO03/009800 ,

En d'autres termes, le comprimé mucoadhésif à libération prolongée selon l'invention permet d'instaurer une concentration salivaire supérieure à 100 µg/ml, de manière continue, sur 24h, ce qui n'est pas observé avec le comprimé de référence ou les autres formes pharmaceutiques habituelles tels que les gels et bains de bouche à base miconazole.

La Demanderesse a également observé que le rapport « dureté/adhésivité » est un paramètre important pour obtenir un profil de libération amélioré du miconazole. De préférence, le rapport « dureté/adhésivité » est supérieur à 0,04 et inférieur à 0,4 dans le comprimé selon l'invention. Enfin, la Demanderesse a montré qu'il est avantageux de préparer le comprimé selon l'invention par granulation par voie humide, comme décrit dans la demande WO03/009800, mais en fractionnant l'ajout d'HPMC de manière à ce qu'environ la moitié de l'HPMC soit présent dans les grains primaires et le reste soit présent en phase externe. Un tel fractionnement permet d'obtenir des comprimés présentant une dureté et une bioadhésivité optimisées. Par ailleurs, ce fractionnement permet de rendre plus robuste le procédé de fabrication, en améliorant la compressibilité du comprimé et en minimisant l'effet de l'étape de compression sur la proportion totale de miconazole libérée lors des tests de dissolution in vitro.

Sans vouloir être liée par une quelconque théorie, la Demanderesse est d'avis que le comprimé mucoadhésif selon l'invention permet d'instaurer des concentrations salivaires très élevées et prolongées d'antifongique, ce qui aurait l'avantage clinique majeur de traiter les candidoses oropharyngées en particulier celles à biofilm ou résistantes aux traitements habituels.

Ainsi, en permettant d'instaurer une concentration salivaire de miconazole nettement supérieure 100 µg/ml pendant sa durée d' application (24h), le comprimé mucoadhésif de miconazole selon l'invention est particulièrement adapté pour le traitement et la prévention des infections oropharyngées à biofilm, contrairement aux formes pharmaceutiques habituelles disponibles dans le commerce.

Ainsi, l'invention a pour objet un comprimé mucoadhésif à libération prolongée caractérisé en ce qu'il comprend de 25 mg à 150 mg de miconazole en tant que principe actif, un concentré de protéines de lait et de l'hydroxy propyl méthyl cellulose en tant qu'excipients et en ce qu'il présente une dureté supérieure à 20 N. De préférence, le comprimé selon l'invention présente une adhésivité supérieure à 100 g et un rapport « dureté/adhésivité » supérieur à 0,04.

Le comprimé selon l'invention est de préférence obtenu par granulation et comprend des grains primaires comprenant le miconazole, les protéines de lait, et une partie de l'HPMC, et une phase externe comprenant le reste de l'HPMC, l'HPMC présent dans les grains primaires représentant de 45% à 55% en poids du poids total de l'HPMC présent dans le comprimé.

Le comprimé selon l'invention est particulièrement adapté pour les sujets souffrant ou prédisposés aux infections oropharyngées à biofilm.

L'utilisation dudit comprimé pour traiter ou prévenir les infection oropharyngées à biofilm est décrite mais ne fait pas partie de la présente invention, notamment les candidoses buccales à biofilm.

Une méthode de traitement d'une infection oropharyngée à biofilm est décrite mais ne fait pas partie de la présente invention, notamment les candidoses buccales à biofilm chez un sujet comprenant l'application d'un comprimé mucoadhésif selon l'invention sur la gencive du patient, ladite application pouvant être renouvelée au bout de 24h de préférence sur une période d'au moins 7 jours.

### 1. Le comprimé mucoadhésif à libération prolongée selon l'invention :

Comme indiqué ci-avant, l'invention a pour objet un comprimé mucoadhésif à libération prolongée de miconazole tel que décrit dans les revendications.

On entend par *« comprimé mucoadhésif »* un comprimé capable d'adhérer sur une muqueuse pendant une durée prolongée, à savoir plusieurs heures. Dans le cadre de la présente invention, la muqueuse se réfère à la muqueuse buccale, de préférence à la gencive. Le comprimé mucoadhésif selon l'invention est capable d'adhérer à la surface de la gencive sur une période d'au moins 18h, de préférence d'au moins 20h, et de manière encore plus préférée sur une période d'au moins 24h.

La route d'administration du comprimé selon l'invention est donc la voie muqueuse, plus précisément la muqueuse buccogingivale.

On entend par *« libération prolongée »* le fait qu'une forme pharmaceutique permet la libération d'un principe actif de manière contrôlée sur une période prolongée, à savoir sur plusieurs heures.

On entend par *« miconazole »* le composé de N° de CAS 22916-47-8 de formule chimique suivante : 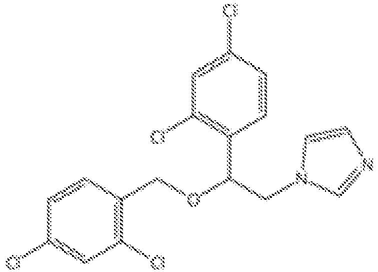 y compris les mélanges racémiques, les énantiomères et les sels acceptables sur le plan pharmaceutique de celui-ci.

Un sel acceptable sur le plan pharmaceutique préféré est le nitrate de miconazole.

Le miconazole est présent dans le comprimé selon l'invention en une quantité allant de 25 mg à 150 mg. De préférence, le miconazole est présent en une quantité de 50 mg ou de 100 mg dans le comprimé selon l'invention.

Comme indiqué ci-avant, le comprimé mucoadhésif selon l'invention se caractérise par une dureté d'au moins 20 N, de préférence allant de 20 à 50 N ou de 25 N à 40 N.

Dans le cadre de la présente, la dureté du comprimé (encore appelée « résistance à l'écrasement) est déterminée de préférence selon la Pharmacopée Européenne § 2.9.8 (European Pharmacopeia 10.0, p. 337, section: Resistance to crushing of tablets).

Le comprimé mucoadhésif selon l'invention se caractérise également par une adhésivité supérieure à 100g, de préférence supérieure à 150 g, par exemple allant de 150 g à 600 g, de 150 g à 500 g, de 200 g à 550 g ou encore de 300 g à 500 g.

Dans le cadre de la présente invention, l'adhésivité (également appelée ici la bioadhésivité, force d'adhésion ou bioadhésion) correspond à la force d'adhésion du comprimé pour une surface donnée, typiquement de l'acier inoxydable. Cette force d'adhésion peut être déterminée à l'aide d'un texturomètre selon une méthodologie classique.

Typiquement, la surface supérieure du comprimé est collée sur la sonde jetable du texturomètre à l'aide d'une colle à base de cyanoacrylate. Le comprimé est ensuite mis en contact avec un support en acier inoxydable pendant un temps donné dans un cristallisoir rempli d'eau purifié. Le bras de levier du texturomètre est ensuite remonté à une vitesse définie et la force nécessaire pour arracher le comprimé est mesurée, permettant à avoir ainsi accès à la force d'adhésivité. De préférence, la force d'adhésion est déterminée selon le protocole fourni dans les exemples. Dans un mode de réalisation supplémentaire, le comprimé mucoadhésif selon l'invention se caractérise par un rapport « dureté/adhésivité » supérieur à 0,04, la dureté étant exprimée en Newton (N) et l'adhésivité en unité d'accélération (g) et les dites dureté et adhésivité étant déterminées selon les méthodes décrites ci-avant.

Dans un mode de réalisation particulier, le rapport « dureté/adhésivité » est compris dans une gamme allant de 0,04 à 0,4, par exemple dans la gamme allant de 0,04 à 0,1.

Selon certains modes de réalisation, le comprimé mucoadhésif à libération prolongée selon l'invention se caractérise par le profil de libération *in vitro* suivant :
- Au plus 45% du miconazole présent dans le comprimé est libéré en 4 heures,
- Au plus 85% du miconazole présent dans le comprimé est libéré en 8 heures, et
- Au moins 85 % du miconazole présent dans le comprimé est libéré en 24h,

Dans certains modes de réalisation additionnels, le comprimé mucoadhésif à libération prolongée selon l'invention se caractérise par le profil de libération *in vitro* suivant
- De 20% à 45% du miconazole présent dans le comprimé est libéré en 4 heures,
- De 60 % à 85% du miconazole présent dans le comprimé est libéré en 8 heures, et
- Au moins 85 % du miconazole présent dans le comprimé est libéré en 24h,

Le profil de dissolution *in vitro* peut être déterminé par un test de dissolution de préférence tel que défini dans la pharmacopée US (USP), chapitre 711 dans les conditions suivantes :
- dispositif de dissolution à paniers : USP Dissolution Apparatus 1
- milieu de dissolution : SDS à 0,5% dans de l'eau purifiée à pH ajusté à 6.5±0,5
- Volume de dissolution: 1000 mL
- température : 37±0,5°C et
- vitesse de rotation : 60 rpm (rotation par minute).

La libération du miconazole dans le milieu est suivie par analyse HPLC avec détection UV (absorbance à 220 nm).

Dans des modes de réalisation additionnels ou alternatifs, le comprimé mucoadhésif selon l'invention est caractérisé en ce qu'il fournit une concentration salivaire moyenne en miconazole à 24h (C₂₄ₕ) d'au moins 100 µg/ml, de préférence au moins 110, 120, 130, ou 140 µg/ml. Typiquement, la C₂₄ₕ moyenne est d'environ 150 µg/ml pour un comprimé comprenant une dose de 50 mg de miconazole et la C₂₄ₕ moyenne est d'environ 425 µg/ml pour un comprimé comprenant une dose de 100 mg de miconazole.

Dans certains modes de réalisation, le comprimé mucoadhésif selon l'invention comprend 50 mg de miconazole et est caractérisé par une Cmax salivaire moyenne de 300 à 500 µg/ml (e.g. environ 350 µg/L), une Tmax salivaire moyenne d'environ 13 à 15h (e.g. environ 14h) et une AUCinf salivaire moyenne de 2000 à 3000 µg.h/mL (e.g. environ 2600 µg.h/mL).

Dans d'autres modes de réalisation, le comprimé mucoadhésif selon l'invention comprend 100 mg de miconazole et est caractérisé par une Cmax salivaire moyenne de 500 à 700 µg/ml (e.g. environ 600 µg/L), une Tmax salivaire moyenne d'environ 15,5 à 17,5h (e.g. environ 16h) et une AUCinf salivaire moyenne de 4 300 à 5 500 µg.h/mL (e.g. environ 4 800 µg.h/mL).

Les paramètres de pharmacocinétiques ci-dessus mentionnés sont des valeurs moyennes obtenues à partir un panel de sujets âgés d'au moins 18 ans et en bonne santé. Le nombre de sujets intégrés dans le panel est choisi de manière à ce que les résultats obtenus soient statistiquement significatifs.

Chaque sujet reçoit un comprimé unique selon l'invention (à 50 ou 100 mg de miconazole) qui est placé au niveau de la gencive supérieure, de préférence au -dessus des incisives. Pour chaque sujet, des prélèvements salivaires sont effectués à intervalles de temps réguliers (typiquement toutes les heures), pendant 24h, afin de doser le miconazole. Pour chaque individu, les différents paramètres de pharmacocinétiques peuvent être déterminés de manière classique à partir de la courbe donnant la concentration salivaire de miconazole en fonction du temps. Les valeurs moyennes C₂₄ₕ, Cₘₐₓ, Tmax, et AUCinf peuvent être déterminées à partir des données brutes des individus par la mise en œuvre de méthodes statistiques bien connues.

Tels qu'utilisés ici, C₂₄ₕ représente la concentration salivaire en miconazole 24h après l'application du comprimé sur la gencive du sujet, Cmax représente la concentration salivaire maximale de miconazole atteinte après l'application du comprimé, Tmax représente le temps au bout duquel la Cmax est atteinte et l'AUCinf représente l'aire sous la courbe retraçant la concentration en miconazole au cours du temps extrapolée à l'infini.

Comme indiqué ci-avant, le comprimé mucoadhésif à libération prolongée se caractérise par la présence d'un concentré de protéines de lait et d'HPMC en tant qu'excipients. Les Inventeurs ont montré que l'utilisation d'un concentré de protéines de lait en combinaison avec l'hydroxypropyl méthyl cellulose en tant qu'excipients a un impact majeur sur les propriétés d'adhésivité du comprimé et le profil de libération du principe actif.

Le concentré de protéines de lait joue un rôle de bioadhésif, assurant l'adhésivité du comprimé sur la muqueuse gingivale.

Il s'agit de préférence d'un concentré de protéines issu de lait de vache. En d'autres termes, il s'agit d'un extrait de protéines de lait de vache au moins partiellement purifiées, c'est à dire présentant une teneur en protéines d'au moins 80%. Les protéines de lait englobent la caséine, l'albumine et les globulines. La caséine est la protéine majoritaire dans le concentré selon l'invention.

Ce concentré de protéines se présente sous la forme d'une poudre et est typiquement obtenu à partir de lait vache par un procédé comprenant typiquement une étape d'écrémage, une étape de pasteurisation, une étape de diafiltration ou d'ultrafiltration et une étape de séchage par atomisation. De préférence, le concentré de protéines de lait a une teneur d'au moins 85%, de préférence d'au moins 86% ou d'au moins 87% en poids de protéines par rapport au poids total sec du concentré. Le concentré de protéines de lait comprend également une teneur en calcium d'au moins 0,5 %, de préférence d'au moins 1,0% ou 1,5 %, par exemple de 1,5% à 3,0% en poids par rapport au poids total du concentré. Par exemple, la teneur en calcium peut aller de 2,0 g à 2,5 g pour 100 g de concentré de protéines de lait.

Sans vouloir être lié par une quelconque théorie, les inventeurs sont d'avis que la présence d'ions calcium participe aux propriétés bioadhésives du concentré de protéines selon l'invention.

Le concentré de protéines de lait est typiquement présent en une teneur allant de 15% à 30% en poids, de préférence de 18% à 28%, par exemple de 20% à 26% en poids par rapport au poids total du comprimé.

L'hydroxyl propyl méthyl cellulose (HPMC) joue à la fois le rôle de liant et de matrice hydrophile participant au gonflement du comprimé et à la libération contrôlés du principe actif et potentialise les effets du concentré de protéines de lait. On peut utiliser de l'HPMC présentant une viscosité apparente allant de 10 000 à 25 000 mPa.s, par exemple de 11 000 à 22 000 cPs , typiquement à 2% dans l'eau à 20°C.

L'HPMC est typiquement présent en une teneur allant de 10% à 25 % en poids, de préférence de 15% à 20% en poids par rapport au poids total du comprimé.

Le comprimé selon l'invention peut comprendre d'autres excipients.

De préférence, il comprend également un surfactant,
Le surfactant peut être utilisé en tant qu'agent mouillant et pour favoriser la dissolution du principe actif. Les surfactants englobent, sans y être limités, la simethicone, la triéthanolamine, les dérivés polysorbate tels que le tween^{®} 20 ou le tween^{®} 40, les poloxamers, les alcools gras tels que l'alcool laurylique ou l'alcool cétylique ou encore les alkylsulfates comme les sels de dodécyl sulfate (également appelé lauryl sulfate). Le surfactant est de préférence le lauryl sulfate de sodium (aussi connu sous le nom de dodécyl sulfate de sodium ou SDS). Il est typiquement présent en une teneur allant de 2% à 8% en poids du poids total du comprimé.

De préférence, le comprimé selon l'invention est obtenu par un procédé de granulation en particulier par voie humide.

Typiquement, et comme cela est détaillé ci-après, le procédé de granulation comprend une étape de préparation de grains primaires, auxquels sont ajoutés dans une étape ultérieure d'autres excipients. Le mélange obtenu est ensuite compressé pour obtenir le comprimé. Les excipients rajoutés dans l'étape ultérieure sont présents en phase externe, donc à l'extérieur des grains primaires.

Ainsi, dans un mode de réalisation particulier, le comprimé selon l'invention est obtenu par granulation et comprend des grains primaires comprenant le miconazole, le concentré de protéines de lait et une partie de l'HPMC, la partie restante de l'HPMC étant en phase externe et représentant de 45 % à 55%, de préférence de 46% à 54%, par exemple de 47% à 53%, de 48% à 52% voire environ 50% en poids du poids total de l'HPMC présent dans le comprimé.

De préférence, le comprimé comprend un surfactant comme le SDS, ledit surfactant étant en phase externe.

Le comprimé selon l'invention peut comprendre en outre un désintégrant, et un lubrifiant.

Le désintégrant peut être utilisé pour améliorer le gonflement du comprimé et/ou la libération du principe actif. Il peut être choisi, par exemple, parmi la polyvinyl pyrrolidone réticulée (crospovidone), la carboxyméthyl cellulose réticulée (telle que la croscarmellose de sodium) ou non-réticulée, les amidons et leurs mélanges. Le désintégrant est de préférence un amidon, par exemple un amidon de maïs. Il est typiquement présent en une teneur allant de 5% à 15% en poids du poids total du comprimé. Le désintégrant est de préférence présent dans les grains primaires, lorsque le comprimé selon l'invention est préparé par granulation.

Le lubrifiant peut être un ou plusieurs composés capables d'empêcher les problèmes liés à la préparation des formes galéniques sèches, comme les problèmes de collage et/ou de grippage qui surviennent au niveau des machines lors du remplissage et/ou de la compression. Les lubrifiants préférés sont des acides gras ou des dérivés d'acides gras comme le stéarate de calcium, le monostéarate de glycéryle, le palmitostéarate de glycéryle, le stéarate de magnésium, le stéarate de zinc, ou l'acide stéarique, les polyalkylèneglycols, notamment le polyéthylèneglycol, le benzoate de sodium ou le talc. Les lubrifiants préférés selon l'invention sont les sels de stéarate et leurs mélanges. Un lubrifiant adapté est par exemple le stéarate de magnésium, le talc et les mélanges de ceux-ci. Le lubrifiant est typiquement présent en une teneur allant de 1% à 3% en poids du poids total du comprimé, et de préférence en phase externe, lorsque le comprimé selon l'invention est préparé par granulation.

A titre optionnel, le comprimé selon l'invention comprend un ou plusieurs excipients choisis parmi un diluant, un arôme, un agent de salivation et les combinaisons de ceux-ci.

Par exemple, le comprimé selon l'invention comprend de 0,1% à 3% en poids d'un diluant ou d'un agent liant de préférence choisi parmi les monosaccharides et les polyols tels que le mannitol, le lactose, le xylose ou galactose, de préférence le lactose.

Dans certains modes de réalisation, le comprimé selon l'invention comprend du lactose, ce lactose étant utilisé en tant qu'agent liant dans le procédé de granulation.

Dans certains modes de réalisation, le comprimé mucoadhésif selon l'invention comprend
- de 35% à 50% de miconazole,
- de 10% à 25 %, de préférence de 15% à 20% d'HPMC
- de 15% à 30%, de préférence de 20% à 26% d'un concentré de protéines de lait,
- de 5% à 15%, de préférence de 6% à 10 % d'un désintégrant, de préférence un amidon de maïs,
- de 0% à 3% de lactose, de préférence de 0,1% à 0,8% de lactose monohydrate,
- de 2% à 8% de surfactant, de préférence de 3% à 6% de dodécylsulfate de sodium, et
- de 1% à 3% de lubrifiant, de préférence de 1,5% à 2,5% de magnésium stéarate et de talc, les pourcentages étant exprimés en poids par rapport au poids total du comprimé.

La dose de miconazole par comprimé est de 25 mg à 150 mg de miconazole, de préférence de 35 mg à 135 mg de miconazole, ou de 40 mg à 110 mg de miconazole, par exemple de 50 ou 100 mg de miconazole.

Dans certains modes de réalisation, le comprimé selon l'invention peut être fabriqué par un procédé de granulation, de préférence par voie humide. De préférence, (i) le ou les lubrifiant(s) (e.g. le talc et le stéarate de magnésium) sont présents en phase externe, (ii) le miconazole, le désintégrant, le concentré de protéines de lait et le lactose sont présents dans les grains primaires et (iii) l'HPMC est réparti entre les grains primaires et la phase externe selon un rapport massique allant de 45/55 à 55/45, de préférence environ 1/1.

Dans un mode de réalisation particulier, le comprimé selon l'invention est fabriqué par un procédé comprenant les étapes suivantes :
a) le mélange du miconazole avec une partie de l'HPMC, le concentré de protéines de lait et le désintégrant
b) la granulation humide du mélange de l'étape a) par ajout d'eau comprenant en option un diluant, de préférence du lactose,
c) le séchage et le calibrage des grains obtenus à l'étape b),
d) l'ajout du reste d'HPMC, du surfactant et des lubrifiants aux grains obtenus à l'étape c), et
e) la compression et la mise en forme du mélange de l'étape d) de manière à obtenir un comprimé.

L'étape de granulation peut être réalisée dans tout type de granulateur, par exemple à granulateur à cisaillement.

L'étape c) est de préférence réalisée de manière à obtenir une teneur en eau comprise entre 3%et 9% en poids, de préférence de 5% à 7% dans les grains. Le séchage peut être réalisé à une température comprise entre 40°C et 60°C, par exemple dans un séchoir à lit mobile.

Une étape de séchage supplémentaire peut être introduite entre l'étape d) et e) de manière à contrôler la teneur en eau finale des comprimés à une valeur inférieure à 8%.

L'étape e) est mise en œuvre par des techniques classiques et de manière à obtenir un comprimé ayant un dureté finale d'au moins 20 N, de préférence de 20 N à 50 N ou de 25 N à 40 N. Typiquement cette étape peut être réalisée avec une presse à comprimés rotative appliquant une force de compression allant de 10 kN à 20 kN.

Sans vouloir être liés par une quelconque théorie, les inventeurs sont d'avis que l'ajout fractionné de l'HPMC aux étapes a) et d) contribuent à améliorer les propriétés physicochimiques et de libération contrôlée du principe actif. Il permet également d'améliorer la robustesse du procédé de fabrication. Typiquement, entre 45% et 55% (e.g. de 46% à 54%, de 47% à 53%, de 48% à 52%, de 49% à 51%), de préférence environ 50% de la quantité finale d'HPMC présente dans le comprimé est ajoutée à l'étape a), le reste étant rajouté à l'étape d). Le procédé peut comprendre des étapes supplémentaires telles que le tamisage et/ou le séchage des différentes matières premières ou encore le conditionnement du comprimé final dans un emballage adapté.

Il va s'en dire que le procédé d'obtention du comprimé tel que décrit précédemment fait aussi partie de l'objet de la présente invention.

### 2. Utilisations et méthodes thérapeutiques selon l'invention : (ces utilisations et méthodes ne font pas partie de l'invention)

Le comprimé mucoadhésif de miconazole selon l'invention est destiné aux sujets souffrant ou susceptible de développer une infection oropharyngée à biofilm.

La présente description divulgue l'utilisation d'un comprimé mucoadhésif tel que décrit ci-avant pour le traitement ou la prévention des infections oropharyngées à biofilm.

Le terme « *traitement* » tel qu'utilisé ici couvre tout traitement d'une maladie chez un sujet d'intérêt, pour : réduire l'incidence et/ou le risque de rechute de la maladie ou du trouble pendant une période sans symptômes; soulager ou réduire un symptôme ou un trouble associé à la maladie; empêcher la maladie ou l'un de ses symptômes ou troubles associés ou le trouble de se produire chez un sujet malade ou prédisposé à la maladie; stopper, ralentir ou décaler dans le temps le développement de la maladie ou de l'un de ses symptômes ou troubles associés; réduire la fréquence et/ou l'intensité des épisode de la maladie, par exemple des symptômes; et soulager la maladie ou un symptôme ou trouble associé, c'est-à-dire provoquer une régression totale ou partielle de ladite maladie, symptôme ou trouble associé.

Le terme de « *prévention* » tel qu'utilisé ici renvoie à un traitement prophylactique visant à empêcher, ralentir ou décaler dans le temps l'apparition d'une maladie ou de l'un de ses symptômes ou troubles associés.

Au sens de l'invention, les infections oropharyngées concernent des infections touchant une ou plusieurs muqueuses de l'oropharynx, notamment de la bouche et de la gorge. Ces infections peuvent impliquer des microorganismes de tout type, notamment des levures ou des bactéries. On parle d'infections à biofilm lorsque l'on observe la présence d'un structure tridimensionnelle composée d'une communauté de microorganismes pathogènes entourée d'une matrice extracellulaire de type polysaccharides protectrice et adhésive se développant au niveau d'une surface de l'oropharynx, par exemple au niveau d'une gencive, du palais ou de la langue.

Dans un mode de réalisation préféré, l'infection oropharyngée est une candidose, de préférence une candidose buccale. On entend par « candidose » une infection causée par une ou plusieurs espèces appartenant au genre *Candida.* Dans certains modes de réalisation, l'infection oropharyngée implique au moins une espèce de *Candida* choisie parmi *C*. *albicans, C. tropicalis, C. glabrata, C. kefyr, C. guilliermondii, C. krusei, C. lusitaniae et C. parapsilosis.*

Le sujet souffrant ou susceptible de souffrir d'une infection oropharyngée à biofilm peut être un homme ou une femme de tout âge.

Dans un mode de réalisation particulier, il s'agit d'un sujet à risque, c'est-à-dire d'un sujet présentant un ou plusieurs facteurs favorisant les infections à biofilm.

Dans un mode de réalisation particulier, le sujet souffrant ou susceptible de souffrir d'une infection oropharyngée à biofilm est choisi parmi les nourrissons, les sujets âgés, de préférence âgés d'au moins 75 ans, les diabétiques, les sujets souffrant d'un cancer ayant notamment subi une radiothérapie notamment au niveau de la tête et du cou ou une chimiothérapie, les sujets souffrant d'hyposialie physiologique ou iatrogène, les sujets sous corticothérapie, notamment par inhalation, les sujets sous traitement antibiotique, les sujets ayant fait une surconsommation de bains de bouche antibactériens, les sujets immunodéprimés, par exemple souffrant du VIH ou sous immunosuppresseurs, les fumeurs, les sujets souffrant de malnutrition ou de troubles alimentaires et les sujets portant un dispositif médical oral.

Dans le cadre de la présente invention, on entend par « *dispositif médical oral* » tout dispositif médical susceptible d'être disposé dans la bouche notamment les prothèses dentaires fixes par exemple les couronnes ou les implants avec pivot tels que les bridge, les prothèses dentaires amovibles, notamment clipsables sur pivot, les dentiers et les appareils d'orthodontie tels que les gouttières, les appareils à brackets ou bagues ou encore les appareils avec une plaque palatine.

Dans certains modes de réalisation, le sujet porte un dispositif médical dentaire.

A titre d'exemple, l'infection oropharyngée à biofilm peut être une stomatite candidosique notamment associée au port d'un dentier ou d'une prothèse dentaire. La stomatite peut être également associée au muguet, notamment à l'intérieur des joues et sur la langue. Il peut s'agir également d'une candidose érythémateuse chronique ou d'une ouranite candidosique.

Dans le cadre des utilisations et traitements thérapeutiques le comprimé mucoadhésif est positionné sur la gencive, par exemple au-dessus d'une incisive ou d'une canine de la mâchoire supérieure. Le comprimé est renouvelé toutes les 24h environ. La dose à administrer (comprimé de 50 ou 100 mg de miconazole) et la durée du traitement dépend des caractéristiques du sujet notamment de son âge, de son poids, de la gravité de l'infection oropharyngée, de son état de santé générale et des comorbidités. La durée du traitement est d'au moins une semaine, généralement de 7 à 14 jours. Ce traitement peut être rallongé ou répété si besoin en fonction de l'état du sujet et de la progression de la maladie.

D'autres aspects et avantages de la présente invention apparaîtront à la lecture des exemples qui suivent, qui doivent être considérés comme illustratifs et en aucun cas comme limitatifs.

### EXEMPLES

### EXEMPLE 1

### Comprimés selon l'invention et comprimés de référence

On a fabriqué des comprimés à 50mg ou 100 mg de miconazole selon l'invention présentant la composition suivante :
- de 43,5% de miconazole,
- de 17,8% d'HPMC
- de 23,9% d'un concentré de protéines de lait ayant une teneur en protéine de 87% et une teneur en Calcium de 2,2% en poids,
- de 8,0 % d'amidon de maïs,
- de 0,3% de lactose,
- de 4,5% de dodécylsulfate de sodium, et
- de 1,15% de magnésium stéarate et de 0,9% de talc,
les pourcentages étant exprimés en poids par rapport au poids total du comprimé

Ces comprimés ont été fabriqués avec la méthodologie suivante :
a) mélange du miconazole avec 50% de l'HPMC, du concentré des protéines de lait, et l'amidon de maïs
b) la granulation humide du mélange de l'étape a) par ajout d'une solution de lactose dans de l'eau purifiée, sous agitation, dans un granulateur par voie humide à cisaillement élevé,
c) le séchage dans un séchoir à lit mobile à une température d'environ 50°C et le calibrage des grains obtenus à l'étape b),
d) l'ajout du reste d'HPMC, du SDS et des lubrifiants aux grains obtenus à l'étape c), et
e) la compression et la mise en forme du mélange de l'étape d) à l'aide d'une presse rotative à comprimés avec une force de compression de 10 à 20 kN.

Les comprimés ainsi obtenus présentent une dureté comprise entre 25N et 40 N et une adhésivité entre 150 g et 500 g.

Des comprimés de référence présentent une composition qualitativement et quantitativement similaire à celle des comprimés selon l'invention, et correspondent aux comprimés décrits dans la demande WO03/009800. Ces comprimés ont été fabriqués selon un procédé analogue à celui des comprimés selon l'invention sauf que l'HPMC est rajouté uniquement à l'étape a), et un concentré de protéines de lait différent est utilisé et l'étape e). La force de compression à l'étape e) a été ajustée de manière à ce que les comprimés de référence présentent une dureté d'environ 18 N. L'adhésivité de ces comprimés est entre 20 et 30 g.

La dureté des comprimés a été déterminée selon la Pharmacopée Européenne § 2.9.8 (version 10.0).

La force d'adhésion a déterminée à l'aide d'un texturomètre selon la méthodologie suivante: Equipement utilisé:
- Un texturomètre: cet appareil est composé d'un banc de test, d'une prise électrique et d'un clavier de pilotage connecté à un ordinateur pour effectuer l'acquisition des données.
- Une sonde plate en nylon à usage unique creusée avec un évidement central vissé sur la partie mobile de l'équipement de texturomètre supérieur (8,1 mm de diamètre et 1 mm de profondeur).
- Une partie inférieure composée d'un cristallisoir de 4 cm de diamètre dont la base est constituée d'une éprouvette en acier inoxydable similaire à la forme du comprimé.

### Réactifs utilisés:

- Eau purifiée : milieu du cristallisoir
- Colle adhésive cyanoacrylate (par exemple Superglue 3 ou équivalent) pour coller le comprimé à la sonde plate.

### Procédure :

On remplit le cristallisoir avec 10 ml d'eau déminéralisée. On fixe un comprimé par le côté plat au centre de la sonde en nylon avec une goutte de colle adhésive cyanoacrylate. On visse la sonde plate en nylon sur la partie mobile du texturomètre. On appuie la sonde sur la surface de l'éprouvette en acier inoxydable. On relève la sonde après 90 secondes (Force appliquée : 1100 g- vitesse de montée : 0,2 mm/s). Le test est répété pour 30 comprimés pris au hasard dans le lot.

Pour chaque mesure effectuée sur un comprimé individuel, la force d'adhésivité est calculée et exprimée en gramme (1 g est 9,81 × 10⁻³ N). Le résultat du test d'adhésivité est la moyenne des 30 valeurs individuelles de la force d'adhésivité.

### Profil de dissolution in vitro

Le profil de dissolution *in vitro* a été déterminé pour chaque lot de comprimé par un test de dissolution tel que défini dans la pharmacopée US (USP), chapitre 711 dans les conditions suivantes :
- nombre d'échantillons : 6
- dispositif de dissolution à paniers : USP Dissolution Apparatus 1
- milieu de dissolution : SDS à 0,5% dans de l'eau purifiée à pH ajusté à 6.5±0,5
- Volume de dissolution: 1000 mL
- température : 37±0,5°C et
- vitesse de rotation : 60 rpm (tour par minute).

La libération du miconazole dans le milieu est suivi par analyse HPLC avec détection UV (absorbance à 220 nm).

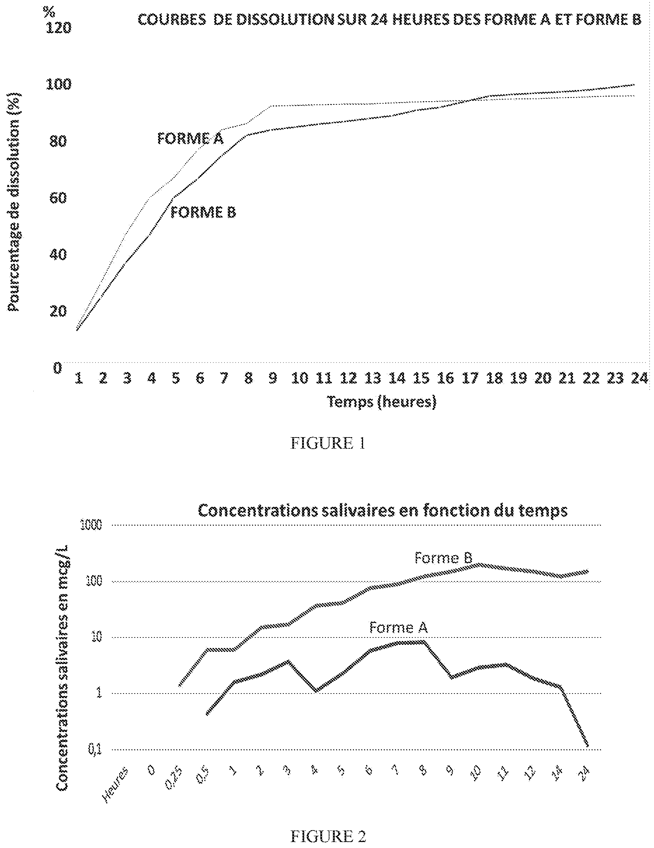

La Figure 1 montre les profils de dissolution obtenus. Les comprimés selon l'invention (forme B ) présentent une libération du miconazole plus prolongée que celle des comprimés de référence.

### Evaluation pharmacocinétique

Les deux lots de comprimés (lot selon l'invention et lot de référence) ont été évalués au cours de deux études cliniques de pharmacocinétique distinctes réalisées à deux moments différents (étude 1 et étude 2). Il s'agit d'études pharmacocinétiques menées auprès de sujets (hommes et femmes) en bonne santé de 18 à 35 ans. Le but de ces études était de déterminer la pharmacocinétique du miconazole salivaire après application une fois par jour d'un comprimé mucoadhésif, 50 ou 100 mg, La pharmacocinétique salivaire et plasmatique du miconazole a été évaluée sur une période de 24 heures.

Pour chaque sujet, un comprimé selon l'invention ou un comprimé de référence a été placé dans la région buccale (gencive supérieure juste au-dessus de la dent incisive) jusqu'à son érosion ou le décollement. Les paramètres évalués sont : la concentration de miconazole dans les échantillons de salive, et dans les échantillons de plasma, le temps nécessaire à l'érosion ou au décollement du comprimé bioadhésif, l'évaluation de la tolérabilité basée sur la déclaration des événements indésirables et l'examen local (buccal). Les concentrations de miconazole ont été évaluées dans la salive en prélevant des échantillons de salive avant l'administration du médicament et à 0,5, 1, 2, 3, 4, 5, 6, 7, 8, 9, 10, 11, 12, 14 et 24h post-dose. Des échantillons de plasma ont été prélevés avant et 0,5, 1, 4, 6, 8, 12 et 24h post-dose. La limite inférieure de quantification du test était de 0,1 ng / ml.

Le tableau 1 ci-dessous reprend les résultats principaux obtenus :

| | Comprimés de référence | Comprimé selon l'invention |
|---|---|---|
| Durée d'adhésivité | 15h | >24h |
| Cmax salivaire moyenne (µg/mL) | 15 | 346 |
| **C24h salivaire moyenne (µg/mL)** | **0.12*** | **148**** |
| AUCinf salivaire moyenne (µg.h/mL) | 55 | 2630 |
| Cmax plasmatique moyenne (ng/mL) | 180 | 2.2 |
| Tmax salivaire moyenne (h) | 7 | 14 |
| * concentrations salivaires non détectables chez 15 sujets sur 18 (83%) | | |
| **concentrations détectables et mesurables chez 100% des sujets | | |

Il est à noter que la demande WO20039800 décrit pour les comprimés de référence à 50 mg une Cmax salivaire de 15 µg/ml, une tmax de 7h et une AUC 0-12h salivaire de 43 µg.h/mL.

Les comprimés ont été bien tolérés par les patients.

Le comprimé selon l'invention offre une concentration salivaire de miconazole plus élevée et plus durable, ce qui devrait permettre d'augmenter l'efficacité thérapeutique notamment pour le traitement de candidoses à biofilm. On observe en même temps une concentration plasmatique plus faible suggérant un passage systémique moins important et donc une incidence moins élevée d'effets secondaires.

La figure 2 montre l'évolution de la concentration salivaire moyenne au cours du temps pour les comprimés selon l'invention (forme B) *versus* celle obtenue avec obtenue le comprimé de référence (forme A). On voit que les comprimés selon l'invention permettent d'instaurer une concentration salivaire stable, plus élevée et plus prolongée que celle du comprimé de référence. De manière surprenante, la dureté élevée du comprimé selon l'invention a amélioré la pharmacocinétique du miconazole *in vivo.*

### EXEMPLE 2

L'effet de la répartition de l'HPMC entre la phase externe et la phase interne a été étudié. Des comprimés ont été préparés selon un procédé identique à celui de l'exemple 1 avec les répartitions d'HPMC en phase interne/phase externe suivantes : 0/100, 25/75, 50/50, 75/25, et 100/0. Une première série de comprimés a été obtenue avec une force de compression élevée (force +). Une seconde série de comprimés a été obtenue avec une force de compression plus faible (force -). Pour chaque répartition d'HPMC, on a comparé la quantité de miconazole libérée à T24h dans le test de dissolution in vitro suivant :
Réalisation de dissolution in vitro de comprimés (50 mg Miconazole) en conditions non sink (dosage par HPLC)
- Milieu Tampon phosphate pH 6,8 : 20 ml, dans un flacon fermé sans agitation et à 37 °C.
- Durée totale 24h
- Prélèvements à 1h, 2h, 3h, 4h, 6h, 8h et 24h
- A chaque temps, la totalité du milieu a été prélevé (sans retournement du flacon) pour dosage, et 20 ml de milieu a été rajouté.

Pour une répartition d'HPMC donnée, on a comparé la quantité moyenne de miconazole libérée à T 24h pour les comprimés obtenus avec la force + et celle pour les comprimés obtenus avec la force -.

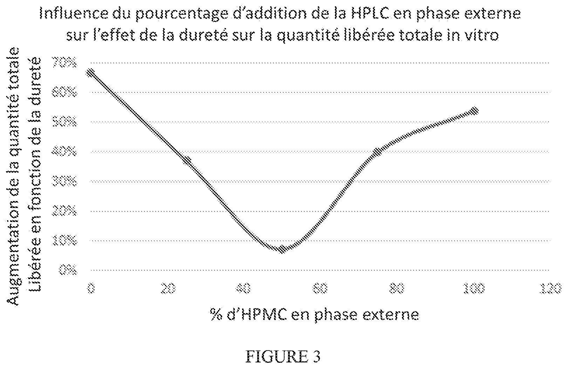

Les résultats sont illustrés dans la Figure 3. La Figure 3 montre que l'effet de la force de compression sur la quantité de miconazole libérée est minimisée lorsque l'HPMC est réparti équitablement en phase externe et en phase interne. On observe alors une variation de 9% entre les comprimés obtenus par compression avec la force + versus les comprimés obtenus avec la force de compression -. En revanche, on note une variation très importante de la quantité libérée lorsque l'HPMC est réparti de manière inégale entre la phase interne et la phase externe.

Une répartition équitable de l'HPMC entre la phase interne et la phase externe permet de minorer l'impact de la force de compression sur la libération du miconazole et donc d'augmenter la robustesse du procédé de fabrication.

## Revendications

1. Comprimé mucoadhésif buccal à libération prolongée comprenant de 25 à 150 mg, de préférence de 35 à 135 mg, de miconazole en tant que principe actif en association avec un concentré de protéines de lait et de l'hydroxypropyl méthylcellulose (HPMC) en tant qu'excipients **caractérisé en ce que** :
- le comprimé présente une dureté supérieure à 20 N et une adhésivité supérieure à 100 g,
- le comprimé est obtenu par un procédé de granulation, de préférence par voie humide,
- le comprimé comprend des grains primaires comprenant le miconazole, le concentré de protéines de lait et une partie de l'HPMC, la partie restante de l'HPMC étant en phase externe des grains primaires et représentant de 45% à 55% en poids du poids total de l'HPMC présent dans le comprimé.

2. Comprimé mucoadhésif selon la revendication 1 **caractérisé en ce que** le comprimé présente un rapport « dureté/adhésivité » supérieur à 0,04, de préférence un rapport « dureté /adhésivité » allant de 0,04 et 0,4, une dureté allant de 25 à 40 N et une adhésivité allant de 150 g à 500 g.

3. Comprimé mucoadhésif selon la revendication 1 ou 2, **caractérisé en ce que**
- le comprimé comprend un surfactant, de préférence un sel de dodecylsulfate, en phase externe, et/ou
- le comprimé comprend un désintégrant tel que l'amidon de maïs présent dans les grains primaires, et un ou plusieurs lubrifiants tels que le talc et le stéarate de magnésium présents en phase externe.

4. Comprimé mucoadhésif selon l'une quelconque des revendications précédentes **caractérisé en ce que** :
- le concentré de protéines de lait se présente sous la forme d'une poudre atomisée et a une teneur en protéine d'au moins 85%, de préférence au moins 87% en poids et une teneur en calcium d'au moins 1% en poids, de préférence d'environ 2,2% en poids et/ou
- l'HPMC a une viscosité apparente dans l'eau à 20°C de 11 000 à 22 000 cPs

5. Comprimé mucoadhésif selon l'une quelconque des revendications précédentes **caractérisé en ce qu'**il présente le profil de libération in vitro suivant:
- De 20% à 45% du miconazole présent dans le comprimé est libéré en 4 heures,
- De 60 % à 85% du miconazole présent dans le comprimé est libéré en 8 heures, et
- Au moins 85 % du miconazole présent dans le comprimé est libéré en 24h,
le profil de dissolution *in vitro* étant déterminé par un test de dissolution de préférence tel que défini dans la pharmacopée US (USP), chapitre 711 dans les conditions suivantes:
- dispositif de dissolution à paniers : USP Dissolution Apparatus 1
- milieu de dissolution : SDS à 0,5% dans de l'eau purifiée à pH ajusté à 6.5±0,5
- Volume de dissolution: 1000 mL
- température : 37±0,5°C et
- vitesse de rotation : 60 rpm.

6. Comprimé mucoadhésif selon l'une quelconque des revendications précédentes, **caractérisé en ce qu'**il instaure une concentration salivaire en miconazole 24h après son application sur la gencive du sujet (C₂₄ₕ) d'au moins 100 µg/mL, de préférence d'au moins 140 µg/mL.

7. Comprimé mucoadhésif selon l'une quelconque des revendications précédentes **caractérisé en ce qu'**il comprend 50 mg de miconazole et présente les propriétés pharmacocinétiques suivantes : une Cmax salivaire moyenne de 300 à 500 µg/, une Tmax salivaire moyenne d'environ 13 à 15h et une AUCinf salivaire moyenne de 2000 à 3000 µg.h/mL.

8. Comprimé mucoadhésif selon l'une quelconque des revendications précédentes **caractérisé en ce qu'**il comprend
- de 35% à 50% de miconazole,
- de 10% à 25 %, de préférence de 15% à 20% d'HPMC
- de 15% à 30%, de préférence de 20% à 26% d'un concentré de protéines de lait,
- de 5% à 15%, de préférence de 6% à 10 % d'un désintégrant, de préférence un amidon de maïs,
- de 0% à 3% de lactose, de préférence de 0,1% à 0,8% de lactose,
- de 2% à 8% de surfactant, de préférence de 3% à 6% de dodécylsulfate de sodium, et
- de 1% à 3% de lubrifiant, de préférence de 1,5% à 2,5% de magnésium stéarate et de talc,
les pourcentages étant exprimés en poids par rapport au poids total du comprimé.

9. Comprimé selon l'une quelconque des revendications précédentes **caractérisé en ce que** la quantité de miconazole est de 50 mg ou 100 mg dans le comprimé.

10. Comprimé selon l'une quelconque des revendications 1 à 9 **caractérisé en ce qu'**il est obtenu par un procédé de granulation humide comprenant les étapes suivantes :
a) le mélange du miconazole avec une partie de l'HPMC, le concentré de protéines de lait et le désintégrant
b) la granulation humide du mélange de l'étape a) par ajout d'eau comprenant le lactose,
c) le séchage et le calibrage des grains obtenus à l'étape b),
d) l'ajout du reste d'HPMC, du surfactant et des lubrifiants aux grains obtenus à l'étape c), et
e) la compression et la mise en forme du mélange de l'étape d) de manière à obtenir un comprimé,
dans lequel de 45% à 55%, de préférence environ 50% en poids du poids total de l'HPMC présentant dans le comprimé final est ajouté à l'étape a).

11. Comprimé mucoadhésif buccal selon l'une quelconque des revendications précédentes pour son utilisation dans le traitement et la prévention d'une infection oropharyngée à biofilm chez un sujet, ladite infection étant de préférence une candidose buccale associée à une ou plusieurs espèces de Candida choisies parmi *C*. *albicans, C. tropicalis, C. glabrata, C. kefyr, C. guilliermondii, C. krusei, C. lusitaniae et C. parapsilosis.*

12. Comprimé mucoadhésif selon l'une quelconque des revendications 1 à 10 pour son utilisation dans le traitement et la prévention d'une infection oropharyngée à biofilm chez un sujet portant un dispositif médical dentaire oral de préférence choisi parmi un dentier, une prothèse dentaire fixe ou amovible et un appareil d'orthodontie.

13. Comprimé mucoadhésif selon l'une quelconque des revendications 1 à 10 pour son utilisation dans le traitement et la prévention d'une infection oropharyngée à biofilm dans laquelle l'infection oropharyngée à biofilm est une stomatite candidosique associée au port d'un dispositif médical dentaire.

14. Comprimé mucoadhésif pour son utilisation selon la revendication 11 dans le traitement et la prévention d'une infection oropharyngée à biofilm, le sujet présentant un ou plusieurs facteurs de risque choisis parmi un âge élevé, de préférence supérieur à 75 ans, le diabète, un cancer, hyposialie physiologique ou iatrogène, une immunodépression, une malnutrition, la consommation de cigarettes, une surconsommation d'antiseptiques buccaux, le port d'un dispositif médical oral, et des traitements médicaux tels qu'une corticothérapie, notamment par inhalation, des antibiotiques, des immunosuppresseurs, une radiothérapie notamment du cou et de la tête et une chimiothérapie.

15. Procédé de fabrication d'un comprimé mucoadhésif de miconazole selon l'une quelconque des revendications 1 à 10 par granulation humide comprenant les étapes suivantes :
a) le mélange du miconazole avec une partie de l'HPMC, le concentré de protéines de lait et le désintégrant
b) la granulation humide du mélange de l'étape a) par ajout d'eau comprenant le lactose,
c) le séchage et le calibrage des grains obtenus à l'étape b),
d) l'ajout du reste d'HPMC, du surfactant et des lubrifiants aux grains obtenus à l'étape c), et
e) la compression et la mise en forme du mélange de l'étape d) de manière à obtenir un comprimé,
dans lequel de 45% à 55%, de préférence environ 50% en poids du poids total de l'HPMC présentant dans le comprimé final est ajouté à l'étape a) et
dans lequel l'étape e) est réalisée de manière à ce que la dureté du comprimé soit d'au moins 20N, de préférence de 20N à 60 N, par exemple de 25N à 50 N.

## Patentansprüche

1. Orale mukoadhäsive Tablette mit verzögerter Freisetzung, umfassend 25 bis 150 mg, vorzugsweise 35 bis 135 mg von Miconazol als Wirkstoff in Kombination mit Milchproteinkonzentrat und Hydroxypropylmethylcellulose (HPMC) als Hilfsstoffen, **dadurch gekennzeichnet, dass**:
- die Tablette eine Härte von mehr als 20 N und eine Klebkraft von mehr als 100 g aufweist,
- die Tablette durch ein Granulierungsverfahren, vorzugsweise nass, gewonnen wird,
- die Tablette Primärkörner umfasst, die Miconazol, das Milchproteinkonzentrat und einen Teil der HPMC umfassen, wobei sich der verbleibende Teil der HPMC in der äußeren Phase der Primärkörner befindet und 45 bis 55 Gew.-% des Gesamtgewichts der in der Tablette vorhandenen HPMC ausmacht.

2. Mukoadhäsive Tablette nach Anspruch 1, **dadurch gekennzeichnet, dass** die Tablette ein "Härte/Klebevermögen"-Verhältnis von mehr als 0,04, vorzugsweise ein "Härte/Klebevermögen"-Verhältnis im Bereich von 0,04 bis 0,4, eine Härte im Bereich von 25 bis 40 Netto und eine Klebefähigkeit im Bereich von 150 g bis 500 g aufweist.

3. Mukoadhäsive Tablette nach Anspruch 1 oder 2, **dadurch gekennzeichnet dass**
- die Tablette in der äußeren Phase ein Tensid, vorzugsweise ein Dodecylsulfatsalz, enthält, und/oder
- die Tablette ein Sprengmittel wie etwa Maisstärke umfasst, die in den Primärkörnern vorhanden ist, und ein oder mehrere Gleitmittel wie etwa Talkum und Magnesiumstearat, die in der äußeren Phase vorhanden sind.

4. Mukoadhäsive Tablette nach einem der vorhergehenden Ansprüche, **dadurch gekennzeichnet, dass**:
- das Milchproteinkonzentrat in Form eines zerstäubten Pulvers vorliegt und einen Proteingehalt von mindestens 85 Gew.-%, vorzugsweise mindestens 87 Gew.-% und einen Calciumgehalt von mindestens 1 Gew.-%, vorzugsweise etwa 2,2 Gew.-%, aufweist und/oder
- das HPMC in Wasser bei 20 °C eine scheinbare Viskosität von 11.000 bis 22.000 cPs hat.

5. Mukoadhäsive Tablette nach einem der vorhergehenden Ansprüche, **dadurch gekennzeichnet, dass** sie das folgende in-vitro-Freisetzungsprofil aufweist:
- 20 % bis 45 % des in der Tablette enthaltenen Miconazols werden innerhalb von 4 Stunden freigesetzt,
- 60 % bis 85 % des in der Tablette enthaltenen Miconazols werden innerhalb von 8 Stunden freigesetzt und,
- mindestens 85 % des in der Tablette enthaltenen Miconazols werden innerhalb von 24 Stunden freigesetzt,
wobei das In-vitro-Auflösungsprofil durch einen Auflösungstest bestimmt wird, vorzugsweise wie im US-amerikanischen Arzneibuch (USP), Kapitel 711, unter den folgenden Bedingungen definiert:
- Korbauflösungsgerät: USP Dissolution Apparatus 1
- Auflösungsmedium: 0,5 % SDS in gereinigtem Wasser bei einem pH-Wert von 6,5 ± 0,5,
- Auflösungsvolumen: 1000 ml
- Temperatur: 37±0,5°C und
- Drehzahl: 60 U/min.

6. Mukoadhäsive Tablette nach einem der vorhergehenden Ansprüche, **dadurch gekennzeichnet, dass** sie 24 Stunden nach ihrer Anwendung auf das Zahnfleisch des Probanden (C24h) eine Miconazolkonzentration im Speichel von mindestens 100 µg/ml, vorzugsweise mindestens 140 µg/ml, aufbaut.

7. Mukoadhäsive Tablette nach einem der vorhergehenden Ansprüche, **dadurch gekennzeichnet, dass** sie 50 mg Miconazol enthält und die folgenden pharmakokinetischen Eigenschaften aufweist: eine durchschnittliche Speichel-Cmax von 300 bis 500 µg/, eine durchschnittliche Speichel-Tmax von etwa 13 bis 15 Stunden und eine durchschnittliche Speichel-AUCinf von 2000 bis 3000 µg.h/ml

8. Mukoadhäsive Tablette nach einem der vorhergehenden Ansprüche, **dadurch gekennzeichnet, dass** sie umfasst
- 35 % bis 50 % Miconazol,
- 10 % bis 25 %, vorzugsweise 15 % bis 20 % HPMC,
- 15 % bis 30 %, vorzugsweise 20 % bis 26 % eines Milchproteinkonzentrats,
- 5 % bis 15 %, vorzugsweise 6 % bis 10 %, eines Sprengmittels, vorzugsweise Maisstärke,
- 0 % bis 3 % Laktose, vorzugsweise 0,1 % bis 0,8 % Laktose,
- 2 % bis 8 % Tensid, vorzugsweise 3 % bis 6 % Natriumdodecylsulfat, und
- 1 % bis 3 % Gleitmittel, vorzugsweise 1,5 % bis 2,5 % Magnesiumstearat und Talk, wobei sich die Prozentangaben auf das Gewicht, bezogen auf das Gesamtgewicht der Tablette beziehen.

9. Tablette nach einem der vorhergehenden Ansprüche, **dadurch gekennzeichnet, dass** die Menge an Miconazol in der Tablette 50 mg oder 100 mg beträgt.

10. Tablette nach einem der Ansprüche 1 bis 9, **dadurch gekennzeichnet, dass** sie durch ein Nassgranulationsverfahren erhalten wird, das die folgenden Schritte umfasst:
a) die Mischung von Miconazol mit einem Teil HPMC, Milchproteinkonzentrat und Sprengmittel
b) die Nassgranulierung der Mischung aus Schritt a) durch Zugabe von lactosehaltigem Wasser,
c) das Trocknen und Kalibrieren der in Schritt (b) erhaltenen Körner,
d) die Zugabe des restlichen HPMC, Tensids und Schmiermittels zu den in Schritt c) erhaltenen Körnern und
e) Verpressen und Formen der Mischung aus Schritt d), um eine Tablette zu erhalten, wobei in Schritt a) 45 bis 55 %, vorzugsweise etwa 50 Gew.-% des Gesamtgewichts des in der fertigen Tablette vorhandenen HPMC hinzugefügt werden.

11. Orale mukoadhäsive Tablette nach einem der vorhergehenden Ansprüche zur Verwendung bei der Behandlung und Vorbeugung einer oropharyngealen Biofilminfektion bei einem Patienten, wobei diese Infektion vorzugsweise eine orale Candidiasis ist, die mit einer oder mehreren Candida-Spezies verbunden ist, ausgewählt aus *C*. *albicans, C. tropicalis, C. glabrata, C. kejj;r, C. guilliermondii, C. krusei, C. lusitaniae* und *C*. *parapsilosis.*

12. Mukoadhäsive Tablette nach einem der Ansprüche 1 bis 10 zur Verwendung bei der Behandlung und Vorbeugung einer oropharyngealen Biofilminfektion bei einem Patienten, der ein zahnmedizinisches Gerät für den Mundraum trägt, das vorzugsweise aus einer Zahnprothese, einer festen oder herausnehmbaren Zahnprothese und einer kieferorthopädischen Vorrichtung ausgewählt wird.

13. Mukoadhäsive Tablette nach einem der Ansprüche 1 bis 10 zur Verwendung bei der Behandlung und Vorbeugung einer oropharyngealen Biofilminfektion, wobei es sich bei der oropharyngealen Biofilminfektion um eine Candida-Stomatitis handelt, die mit dem Tragen eines zahnmedizinischen Geräts verbunden ist.

14. Mukoadhäsive Tablette zur Verwendung gemäß Anspruch 11 bei der Behandlung und Vorbeugung einer oropharyngealen Biofilminfektion, wobei das Subjekt einen oder mehrere Risikofaktoren aufweist, ausgewählt aus hohem Alter, vorzugsweise über 75 Jahre, Diabetes, Krebs, physiologischer oder iatrogener Hyposialie, Immunsuppression, Unterernährung, Zigarettenkonsum, übermäßigem Konsum oraler Antiseptika, Tragen eines oralen medizinischen Geräts und medizinischen Behandlungen wie Kortikosteroidtherapie, insbesondere durch Inhalation, Antibiotika, Immunsuppressiva, Strahlentherapie insbesondere von Hals und Kopf und Chemotherapie.

15. Verfahren zur Herstellung einer mucoadhäsiven Tablette von Miconazol nach einem der Ansprüche 1 bis 10 durch Nassgranulation, das die folgenden Schritte umfasst:
a) die Mischung von Miconazol mit einem Teil HPMC, Milchproteinkonzentrat und Sprengmittel
b) die Nassgranulierung der Mischung aus Schritt a) durch Zugabe von lactosehaltigem Wasser,
c) das Trocknen und Kalibrieren der in Schritt (b) erhaltenen Körner,
d) die Zugabe des restlichen HPMC, Tensids und Schmiermittels zu den in Schritt c) erhaltenen Körnern und
e) Verpressen und Formen der Mischung aus Schritt d), um eine Tablette zu erhalten, wobei in Schritt a) 45 bis 55 %, vorzugsweise etwa 50 Gew.-% des Gesamtgewichts des in der fertigen Tablette vorhandenen HPMC zugefügt werden und
wobei Schritt e) so durchgeführt wird, dass die Härte der Tablette mindestens 20 N, vorzugsweise 20 N bis 60 N, beispielsweise 25 N bis 50 N beträgt.

## Claims

1. A sustained-release buccal mucoadhesive tablet comprising from 25 to 150 mg, preferably from 35 to 135 mg, of miconazole as active principle in combination with a milk protein concentrate and hydroxypropylmethylcellulose (HPMC) as excipients, **characterized in that**:
- the tablet has a hardness of greater than 20 N and an adhesiveness of greater than 100 g,
- the tablet is obtained via a granulation process, preferably by wet granulation,
- the tablet comprises primary grains comprising miconazole, the milk protein concentrate and some of the HPMC, the remainder of the HPMC being in the outer phase of the primary grains and representing from 45% to 55% by weight relative to the total weight of the HPMC present in the tablet.

2. The mucoadhesive tablet of claim 1, **characterized in that** the tablet has a "hardness/adhesiveness" ratio greater than 0.04, preferably a "hardness/adhesiveness" ratio ranging from 0.04 to 0.4, a hardness ranging from 25 to 40 N, and an adhesiveness ranging from 150 g to 500 g.

3. The mucoadhesive tablet of claim 1 or 2, **characterized in that**:
- the tablet comprises a surfactant, preferably a dodecyl sulfate salt, in the outer phase and/or
- the tablet comprises a disintegrant such as corn starch present in the primary grains, and one or more lubricants such as talc and magnesium stearate present in the outer phase.

4. The mucoadhesive tablet of any one of the preceding claims, **characterized in that**:
- the milk protein concentrate is in the form of an atomized powder and has a protein content of at least 85%, preferably at least 87% by weight and a calcium content of at least 1% by weight, preferably about 2.2% by weight and/or
- the HPMC has an apparent viscosity in water at 20°C of from 11 000 to 22 000 cPs.

5. The mucoadhesive tablet of any one of the preceding claims, **characterized in that** it has the following *in vitro* release profile:
- from 20% to 45% of the miconazole present in the tablet is released within 4 hours,
- from 60% to 85% of the miconazole present in the tablet is released within 8 hours, and
- at least 85% of the miconazole present in the tablet is released within 24 hours,
the *in vitro* dissolution profile being determined by a dissolution test preferably as defined in the US Pharmacopea (USP), chapter 711, under the following conditions:
- basket dissolution device: USP Dissolution Apparatus 1
- dissolution medium: 0.5% SDS in purified water with pH adjusted to 6.5 ± 0.5
- Dissolution volume: 1000 mL
- temperature: 37 ± 0.5°C and
- rotation speed: 60 rpm.

6. The mucoadhesive tablet of any one of the preceding claims, **characterized in that** it establishes a salivary concentration of miconazole 24 hours after its application to the subject's gums (C₂₄ₕ) of at least 100 µg/mL, preferably at least 140 µg/mL.

7. The mucoadhesive tablet of any one of the preceding claims, **characterized in that** it comprises 50 mg of miconazole and has the following pharmacokinetic properties: a mean salivary Cmax from 300 to 500 µg/ml, a mean salivary Tmax of about 13 to 15h and a mean salivary AUCinf from 2000 to 3000 µg.h/mL.

8. The mucoadhesive tablet of any one of the preceding claims, **characterized in that** it comprises:
- from 35% to 50% of miconazole,
- from 10% to 25%, preferably from 15% to 20%, of HPMC,
- from 15% to 30%, preferably from 20% to 26%, of a milk protein concentrate,
- from 5% to 15%, preferably from 6% to 10%, of a disintegrant, preferably a corn starch,
- 0% to 3% of lactose, preferably from 0.1% to 0.8% of lactose,
- from 2% to 8% of surfactant, preferably from 3% to 6% sodium dodecyl sulfate, and
- from 1% to 3% of lubricant, preferably from 1.5% to 2.5% of magnesium stearate and talc, the percentages being expressed on a weight basis relative to the total weight of the tablet.

9. The tablet of any one of the preceding claims, **characterized in that** the amount of miconazole in the tablet is 50 mg or 100 mg.

10. The tablet of any one of claims 1 to 9, **characterized in that** it is obtained by a wet granulation process comprising the following steps:
a) mixing miconazole with some of the HPMC, the milk protein concentrate and the disintegrant,
b) wet granulation of the mixture from step a) by adding water comprising the lactose,
c) drying and calibrating the grains obtained in step b),
d) adding the remaining HPMC, the surfactant and the lubricants to the grains obtained in step c), and
e) compressing and shaping the mixture from step d) so as to obtain a tablet,
in which from 45% to 55%, preferably about 50% by weight relative to the total weight of the HPMC present in the final tablet is added in step a).

11. The mucoadhesive tablet of any one of preceding claims, for use in the treatment and the prevention of a biofilm oropharyngeal infection, said infection being preferably a buccal candidiasis associated with one or more *Candida* species chosen from *C*. *albicans, C. tropicalis, C. glabrata, C. kefyr, C. guilliermondii, C. krusei, C. lusitaniae* and *C. parapsilosis.*

12. The mucoadhesive tablet of any one of claims 1 to 10, for use in the treatment and the prevention of a biofilm oropharyngeal infection in a subject wearing an oral dental medical device preferably chosen from dentures, a fixed or removable dental prosthesis and an orthodontic appliance.

13. The mucoadhesive tablet of any one of claims 1 to 10, for use in the treatment and the prevention of a biofilm oropharyngeal infection, in which the biofilm oropharyngeal infection is candidal stomatitis associated with the wearing of a dental medical device.

14. The mucoadhesive tablet of claim 11, for use in the treatment and the prevention of a biofilm oropharyngeal infection, the subject presenting with one or more risk factors selected from high age, preferably over 75 years old, diabetes, cancer, physiological or iatrogenic hyposialia, immunodepression, malnutrition, cigarette smoking, overuse of buccal antiseptics, wearing of an oral medical device, and medical treatments such as corticosteroid therapy, notably by inhalation, antibiotics, immunosuppressants, radiotherapy notably of the neck and head, and chemotherapy.

15. A process for manufacturing a miconazole mucoadhesive tablet of any one of claims 1 to 10 by wet granulation, comprising the following steps:
a) mixing miconazole with some of the HPMC, the milk protein concentrate and the disintegrant,
b) wet granulation of the mixture from step a) by adding water comprising the lactose,
c) drying and calibrating the grains obtained in step b),
d) adding the remaining HPMC, the surfactant and the lubricants to the grains obtained in step c), and
e) compressing and shaping the mixture from step d) so as to obtain a tablet,
in which from 45% to 55%, preferably about 50% by weight relative to the total weight of the HPMC present in the final tablet is added in step a) and
in which step e) is performed so that the hardness of the tablet is at least 20 N, preferably from 20 N to 60 N, for example from 25 N to 50 N.
